# EUROPÄISCHE PATENTSCHRIFT

(11) **EP 2 760 643 B1**
(45) Veröffentlichungstag und Bekanntmachung des Hinweises auf die Patenterteilung: **05.07.2017**
(21) Anmeldenummer: 12766440.7
(22) Anmeldetag: 26.09.2012
(51) Int. Cl.: B25J 15/04

(54) **HANDHABUNGSEINRICHTUNG SOWIE VERFAHREN ZUM BETREIBEN EINER HANDHABUNGSEINRICHTUNG**
HANDLING DEVICE AND METHOD FOR OPERATING A HANDLING DEVICE
APPAREIL DE MANIPULATION AINSI QUE PROCÉDÉ POUR FAIRE FONCTIONNER UN APPAREIL DE MANIPULATION

(30) Priorität: 28.09.2011 DE 102011115077
(43) Veröffentlichungstag der Anmeldung: 06.08.2014
(73) Patentinhaber: Deutsches Zentrum für Luft- und Raumfahrt e.V., 51147 Köln (DE)
(72) Erfinder: FRÖHLICH, Florian, Alexander, 82110 Germering (DE); JÖRG, Stefan, 81241 München (DE)
(74) Vertreter: dompatent von Kreisler Selting Werner - Partnerschaft von Patent- und Rechtsanwälten mbB
(86) Internationale Anmeldenummer: PCT/EP2012/068955
(87) Internationale Veröffentlichungsnummer: WO 2013/045489

(56) Entgegenhaltungen:
- JP-A- 2009 117 732
- US-A1- 2007 142 824
- US-A1- 2011 023 650

## Beschreibung

Die Erfindung betrifft eine Handhabungseinrichtung sowie ein Verfahren zum Verwenden unterschiedlicher Instrumente mit einer Aufnahmevorrichtung einer Handhabungseinrichtung.

Handhabungseinrichtungen, wie Roboter, weisen, beispielsweise an Roboterarmen, Aufnahmevorrichtungen zur Aufnahme von Instrumenten, wie Werkzeugen, auf. Handelt es sich beispielsweise um einen in der Medizin, insbesondere der Chirurgie, eingesetzten Roboter, werden von den Aufnahmevorrichtungen chirurgische Instrumente aufgenommen. Hierbei ist es möglich, dass eine Aufnahmevorrichtung unterschiedliche Instrumente aufnimmt. Dies erfolgt durch einen Instrumente- oder Werkzeugwechsel.

Um sicherzustellen, dass zu einem gewünschten Zeitpunkt das gewünschte Instrument mit der Aufnahmevorrichtung verbunden ist, können an den Instrumenten passive Markierungen, wie Beschriftungen oder dgl., vorgesehen sein. Mit Hilfe einer Arbeitsvorschrift bzw. eines klar definierten Ablaufplans wird das geforderte, entsprechend markierte bzw. bezeichnete Instrument von einem Arbeiter mit der Aufnahmevorrichtung verbunden. Bei Medizinrobotern erfolgt das Verbinden eines chirurgischen Instruments hierbei von einem Operationsassistenten nach Vorgabe eines Operationsplans oder auf Anweisung des operierenden Chirurgen. Dieses Verfahren ist fehleranfällig und ferner nicht flexibel.

Um ein falsches Verbinden von Instrumenten mit einer Aufnahmevorrichtung zu vermeiden, ist es ferner möglich, eindeutige Schnittstellen vorzusehen. Hierbei sind die Schnittstellen der Komponenten derart ausgestaltet, dass nur erwünschte Verbindungen möglich sind. Die Schnittstellen weisen somit einen Mechanismus auf, der ein falsches Verbinden verhindert. Dieses Verfahren ist unflexibel und weist nur eine stark begrenzte Anzahl an Verbindungsmöglichkeiten auf und kann insbesondere nur mit großem Aufwand verändert werden.

Ferner ist es bei Werkzeugmaschinen bekannt, einen automatischen Werkzeugwechsel vorzusehen. Hierbei werden die unterschiedlichen Werkzeuge bzw. Instrumente in einem Magazin bereitgestellt. In einer Steuereinrichtung der Werkzeugmaschine ist ein definierter Ablaufplan vorgegeben. Auf Basis des Ablaufplans erfolgt ein automatischer Werkzeugwechsel. Dies erfolgt dadurch, dass der Werkzeugmaschine bekannt ist, an welcher Position des Magazins sich welches Werkzeug befindet. Ein derartiger automatischer Werkzeugwechsel ist nur mit aufwändigen Vorrichtungen realisierbar. Dies ist beispielsweise beim Instrumentenwechsel bei einem Medizinroboter nicht möglich.

Aus US 2007/0142824 ist ein Medizinroboter mit unterschiedlichen Instrumenten und mehreren Aufnahmevorrichtungen bekannt, wobei entweder an den Instrumenten oder an den Aufnahmevorrichtungen Indikatoreinrichtungen vorgesehen sind, die mittels einer Steuereinrichtung veränderbar sind. Aufgabe der Erfindung ist es, eine Handhabungseinrichtung zu schaffen, bei der auf einfache Weise unterschiedliche Instrumente gewechselt werden können, wobei das Auftreten von Fehlern vermieden werden soll. Ferner ist es Aufgabe der Erfindung, ein Verfahren zum Betreiben einer derartigen Handhabungseinrichtung zu schaffen.

Die Lösung der Aufgabe erfolgt durch eine Handhabungseinrichtung gemäß Anspruch 1 bzw. ein Verfahren gemäß Anspruch 7.

Die erfindungsgemäße Handhabungseinrichtung, bei der es sich insbesondere um einen Roboter, wie einen vorzugsweise in der Chirurgie einsetzbaren Medizinroboter, handelt, weist mindestens eine Aufnahmevorrichtung auf. Die Aufnahmevorrichtung, die beispielsweise an einem insbesondere mehrgelenkigen Roboterarm angeordnet ist, dient zur Aufnahme unterschiedlicher Instrumente. Mit Hilfe der Aufnahmevorrichtung können somit insbesondere zu unterschiedlichen Zeitpunkten unterschiedliche Instrumente, wie Werkzeuge, chirurgische Instrumente und dgl., verbunden werden. Selbstverständlich ist es auch möglich, mit der Aufnahmevorrichtung Zwischenelemente zu verbinden, mit denen sodann Instrumente verbunden werden. Zum Verbinden unterschiedlicher Instrumente mit der mindestens einen Aufnahmevorrichtung weisen die Aufnahmevorrichtung und die Instrumente miteinander kompatible Verbindungselemente auf. Bei den Verbindungselementen handelt es sich um mechanische und ggf. auch elektrische Verbindungselemente, wie Stecker und dgl.

Erfindungsgemäß sind an den Instrumenten veränderbare bzw. aktivierbare Indikatoreinrichtungen vorgesehen. Bei den Indikatoreinrichtungen handelt es sich beispielsweise um Leuchtmittel, wie LEDs, ein Display oder dgl. Mit Hilfe einer Steuereinrichtung kann die Indikatoreinrichtung verändert werden. Hierzu ist die Steuereinrichtung über elektrische Leitungen, Funk oder dgl. mit den Indikatoreinrichtungen der Instrumente verbunden. Insbesondere steuert die Steuereinrichtung in Abhängigkeit einer Steuervorgabe die Indikatoreinrichtung. Bei der Steuervorgabe kann es sich um eine aktuelle Eingabe eines Benutzers der Handhabungsvorrichtung, wie eines Chirurgen, handeln. Ferner kann ein Ablaufplan in der Steuereinrichtung hinterlegt sein, der sodann beispielsweise in Abhängigkeit eines zeitlichen Ablaufs Steuersignale für die Indikatoreinrichtungen erzeugt. Ein Instrumentenwechsel und somit die Veränderung einer Indikatoreinrichtung kann selbstverständlich auch in Abhängigkeit definierter Ereignisse, wie das Erreichen einer bestimmten Instrumentenposition, das Bestätigen eines fertiggestellten Arbeitsschritts durch einen Chirurgen oder dgl. erfolgen und hierbei beispielsweise mit einem Ablaufplan kombiniert werden.

Handelt es sich bei der Indikatoreinrichtung an den unterschiedlichen Instrumenten beispielsweise um eine oder mehrere Leuchtmittel, wie LEDs, so erfolgt durch die Steuereinrichtung beispielsweise ein Indizieren eines gewünschten Instruments durch Aktivieren des Leuchtmittels. Ein Benutzer, wie ein Operationsassistent, verbindet sodann das indizierte gewünschte Instrument mit der Aufnahmevorrichtung. Hierbei ist es aufgrund des aktivierten Leuchtmittels eindeutig, welches Instrument mit der Aufnahmevorrichtung verbunden werden soll.

Zusätzlich ist erfindungsgemäß auch an der Aufnahmevorrichtung eine veränderbare Indikatoreinrichtung vorgesehen. Hierbei wird durch die Steuereinrichtung sowohl die Indikatoreinrichtung an dem gewünschten Instrument als auch an der Aufnahmevorrichtung bei einem gewünschten Instrumentenwechsel aktiviert. Für den Benutzer ist somit nicht nur eindeutig, welches Instrument genutzt werden soll sondern auch mit welcher Aufnahmevorrichtung dieses Instrument verbunden werden soll. Dies ist insbesondere vorteilhaft, wenn eine Handhabungseinrichtung mehrere Aufnahmevorrichtungen aufweist oder mehrere Handhabungseinrichtungen in unmittelbarer Nähe zueinander angeordnet sind. Hierbei kann die Steuerung mehrerer Handhabungseinrichtungen durch eine gemeinsame Steuereinrichtung erfolgen.

Zur weiteren Erhöhung der Sicherheit ist es möglich, dass ein Instrument durch die Steuereinrichtung aktiviert wird. Hierzu weist das Instrument eine entsprechende Aktivierungseinrichtung auf, bei der es sich um eine elektronische Schaltung oder ein elektronisches Bauteil handeln kann. Hierdurch kann beispielsweise sichergestellt werden, dass nur ein derart aktiviertes Instrument mit der Aufnahmevorrichtung verbunden werden kann. Beispielsweise kann die Aktivierung auch derart erfolgen, dass aus einem Instrumentenhalter, in dem die nicht benötigten Instrumente bevorratet sind, ein Instrument nur entnommen werden kann, wenn es aktiviert ist. Beispielsweise kann auch ein mechanisches Aktivierungsmittel vorgesehen sein. Hierbei kann es sich um einen Pin, einen Stift oder dgl. handeln, der beispielsweise nur bei einem aktivierten Instrument betätigt oder verschoben werden kann.

Insbesondere erfolgt vorzugsweise ein Vorsehen aktivierbarer Aktivierungselemente an den Verbindungselementen der Aufnahmevorrichtung und/ oder der unterschiedlichen Instrumente. Die Aktivierung erfolgt über eine Steuereinrichtung. Bei den Aktivierungselementen kann es sich um mechanische oder auch elektronische Aktivierungselemente handeln. Mechanische Aktivierungselemente sind insbesondere Pins, Ansätze und dergleichen, die beispielsweise nur in aktiviertem Zustand bewegt werden können. Als elektronische Aktivierungselemente kann beispielsweise der Austausch oder die Abfrage von Codes oder Schlüsseln erfolgen.

Bei einer bevorzugten Weiterbildung der Erfindung ist die Steuereinrichtung derart ausgebildet, dass ein Ablaufplan hinterlegbar ist, in dem die zeitliche Abfolge der Instrumentenwechsel festgelegt ist. Bei einem derartigen Ablaufplan kann es sich beispielsweise um einen geplanten Operationsablauf handeln. Dieser wird im Vorfeld der Operation festgelegt. In Abhängigkeit des aktuellen Operationsfortschrittes erfolgt sodann über die Steuereinrichtung ein Aktivieren der entsprechenden Indikatoreinrichtung des als nächstes benötigten Instruments. Ggf. kann die Steuereinrichtung zusätzlich oder anstelle eines hinterlegten Ablaufplans eine Eingabeeinrichtung aufweisen bzw. mit einer Eingabeeinrichtung verbunden sein. Über eine derartige Eingabeeinrichtung kann ein gewünschter Instrumentenwechsel aktiviert werden. Bei einem Medizinroboter kann dies beispielsweise durch Eingabe eines Chirurgen erfolgen. Auch kann über die Eingabeeinrichtung eine Bestätigung erfolgen, dass ein gewisser Arbeitsschritt abgeschlossen ist und somit von der Steuereinrichtung der nächste Instrumentenwechsel initiiert wird.

Bei dem erfindungsgemäßen Verfahren zum Verbinden von Instrumenten mit einer Aufnahmevorrichtung einer Handhabungseinrichtung wie vorstehend beschrieben, werden von einer Steuereinrichtung eine an dem entsprechenden Instrument angeordnete sowie eine an der Aufnahmevorrichtung vorgesehene Indikationseinrichtung aktiviert. Die Aktivierung erfolgt über die Steuereinrichtung auf Basis eines hinterlegten Ablaufplans und/ oder mittels einer mit der Steuereinrichtung verbundenen Eingabeeinrichtung. Der Wechsel des Instruments erfolgt sodann beispielsweise durch einen Benutzer, wie einen Operationsassistenten, oder ggf. auch automatisch.

Das Verfahren ist insbesondere, wie vorstehend anhand der Handhabungseinrichtung beschrieben, vorteilhaft weitergebildet.

Die erfindungsgemäße Handhabungseinrichtung sowie das erfindungsgemäße Verfahren sind in besonders bevorzugter Ausführungsform bei Medizinrobotern anwendbar. Auch bei anderen Robotern sind die Handhabungseinrichtung sowie das entsprechende Verfahren vorteilhaft. Insbesondere bei manuell geführter, roboterunterstützter Montage von Modulen sind die erfindungsgemäße Handhabungseinrichtung sowie das entsprechende Verfahren vorteilhaft einsetzbar. Besonders bevorzugt ist ein entsprechender Einsatz derartiger Roboter bei der Montage von Modulen im Weltall, da hier unter Extrembedingungen, insbesondere hohem Zeit- und Kostendruck, gearbeitet werden muss.

Nachfolgend wird die Erfindung anhand bevorzugter Ausführungsformen unter Bezugnahme auf die Zeichnungen näher erläutert.

Es zeigen:
- Fig. 1: eine Prinzipskizze eines Werkzeugwechsels bei einer Handhabungsvorrichtung,
- Fig. 2: eine Prinzipskizze eines Wechsels chirurgischer Instrumente bei einem Medizinroboter,
- Fig. 3a - 3c: unterschiedliche Ausführungsformen der Ausgestaltung elektronischer Aktivierungselemente, und
- Fig. 4: eine schematische Darstellung einer Ausführungsform einer mechanischen Ausgestaltung von Aktivierungselementen.

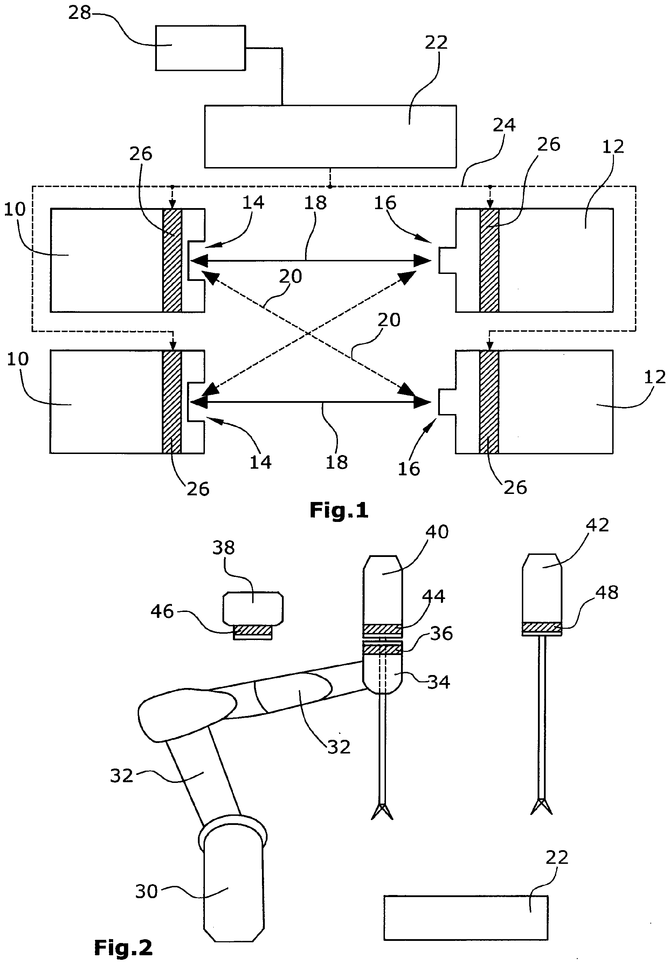

Die Prinzipskizze (Fig. 1) zeigt als Beispiel zwei Komponenten 10, die mit zwei Komponenten 12 verbunden werden können. Hierbei handelt es sich beispielsweise bei den Komponenten 10 um Aufnahmevorrichtungen und bei den Komponenten 12 um Instrumente, wie Werkzeuge, chirurgische Instrumente, Zwischenelemente oder dgl. Die Aufnahmeelemente können hierbei insbesondere mit einem Roboter verbunden sein. Die Komponenten 10 weisen als Ausnehmungen 14 dargestellte Verbindungselemente auf, die mit an den Komponenten 12 als Ansätze schematisch dargestellten Verbindungselemente 16 verbunden werden können. Hierbei ist es möglich, eine der beiden Komponenten 10 mit jeweils einer der beiden Komponenten 12 zu verbinden. Die Verbindungselemente-Paare 14, 16 sind hierbei kompatibel. Es sind somit Verbindungen einer Komponente 10 mit einer Komponente 12, wie durch die Pfeile 18 oder 20 dargestellt, möglich.

Ferner weist die Handhabungseinrichtung eine Steuereinrichtung 22 auf, die über Leitungen 24 oder auch drahtlos mit Indikatoreinrichtungen 26 verbunden sind. Im dargestellten Ausführungsbeispiel ist je Komponente 10, 12 eine Indikatoreinrichtung 26 vorgesehen, bei der es sich beispielsweise um Leuchtmittel, ein Display oder dgl. handelt.

Erfindungsgemäß sind die Indikatoreinrichtungen 26 durch die Steuereinrichtung 22 aktivierbar. Die Aktivierung erfolgt durch ein in der Steuereinrichtung 22 hinterlegtes Ablaufprogramm oder über eine mit der Steuereinrichtung 22 verbundene Eingabeeinrichtung 28, die beispielsweise eine Tastatur, einen Joystick oder dgl. aufweist.

Sofern aufgrund einer Eingabe in die Eingabeeinrichtung 28 und/ oder eines hinterlegten Ablaufplans eine Verbindung der in Fig. 1 oberen Komponente 10 mit der oberen Komponente 12 gewünscht ist, steuert die Steuereinrichtung 22 die beiden entsprechenden Indikatoreinrichtungen 26 derart an, dass diese beispielsweise in der gleichen Farbe leuchten. Ein Bediener der Handhabungseinrichtung weiß somit zweifelsfrei, welche Komponenten zur Durchführung des nächsten Arbeitsschrittes miteinander verbunden werden. Eine Verbindung, wie beispielsweise durch die gestrichelten Pfeile 20 dargestellt, ist nicht gewünscht und erfolgt nicht. Dies kann zusätzlich durch Vorsehen einer nicht dargestellten Aktivierungseinrichtung unterstützt werden, die ein Verbinden von Komponenten, wie durch die Pfeile 20 dargestellt, vermeidet.

Entsprechend kann ein Medizinroboter, wie schematisch in Fig. 2 dargestellt, ausgebildet sein. Hierbei ist eine Basis 30 eines Roboters mit einem mehrgelenkigen Roboterarm 32 verbunden, der an einem distalen Ende mit einer Aufnahmevorrichtung 34 verbunden ist. Diese ist entsprechend der beiden Komponenten 10, 12 (Fig. 1) aufgebaut und weist ein nicht dargestelltes Verbindungselement sowie eine Indikatoreinrichtung 36 auf. Die Aufnahmeeinrichtung 34 kann im dargestellten Ausführungsbeispiel mit drei unterschiedlichen chirurgischen Instrumenten 38, 40, 42 verbunden werden. Soll beispielsweise das vorhandene Instrument 40 durch das Instrument 38 ausgetauscht werden, wird die Indikatoreinrichtung 44 des Instruments 40 deaktiviert und die Indikatoreinrichtung 46 des Instruments 48 aktiviert. Bei einer farblichen Indikation leuchtet die Indikatoreinrichtung 36 der Aufnahmevorrichtung in derselben Farbe wie die Indikatoreinrichtung 46 des Instruments 38.

Die entsprechende Ansteuerung der Indikatorelemente erfolgt über eine Steuereinrichtung 22, die, wie anhand Fig. 1 erläutert, ebenfalls mit einer Eingabeeinrichtung 28 verbunden sein kann. Die Steuereinrichtung 22 steuert somit in Abhängigkeit eines Ablaufplans, der beispielsweise durch den Operationsfortschritt beeinflusst oder aktiviert wird, die Indikationseinrichtungen 44, 46, 48 der chirurgischen Instrumente 38, 40, 42 in Kombination mit der Indikatoreinrichtung 36 der Aufnahmevorrichtung 34. Bei einem Robotersystem mit mehreren Roboterarmen und insofern auch mehreren Aufnahmevorrichtungen, ist das Vorsehen der Indikatoreinrichtung an der Aufnahmevorrichtung zweckmäßig um sicherzustellen, dass das Instrument mit der richtigen gewünschten Aufnahmevorrichtung verbunden wird.

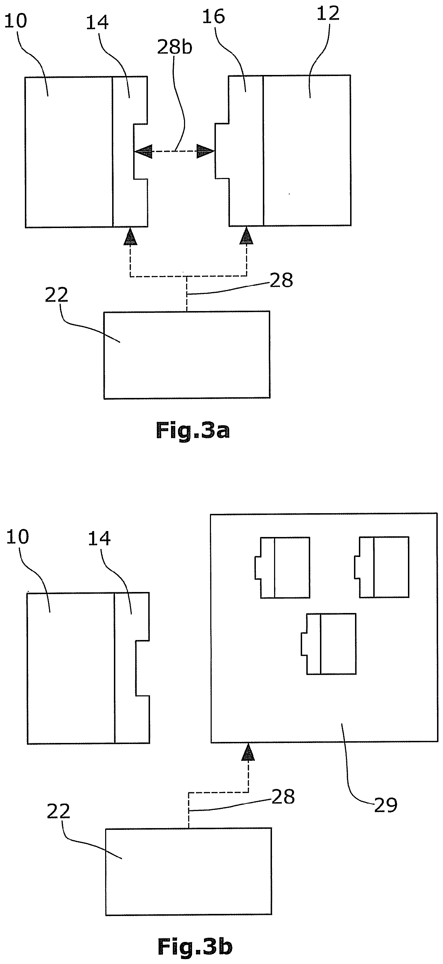

In den Abbildungen 3a - 3c sind unterschiedliche Ausgestaltungen von durch eine Steuereinrichtung 22 aktivierbaren Aktivierungseinrichtungen dargestellt. In allgemeiner Ausführung kommuniziert die Steuereinrichtung 22 mit der Aktivierungseinrichtung 14, welche sich am Verbindungselement des Roboters 10 befindet und mit der

Aktivierungseinrichtung 16, welche sich am Instrument befindet. Ein sicherer Werkzeugwechsel gemäß der Erfindung läuft wie folgt ab: Die Steuereinrichtung aktiviert das gewünschte Werkzeug mit Hilfe der Aktivierungseinrichtung 16 und teilt der Aktivierungseinrichtung 14 am Roboter mit, welches Werkzeug aktiviert wurde. Beim Verbinden der Verbindungselemente erkennen die Aktivierungselemente, ob sie zusammenpassen oder nicht. Nur Verbindungselemente mit passenden Aktivierungselementen lassen sich verbinden. Ob eine Verbindung zustande kommt wird der Steuereinrichtung durch das Aktivierungselement 14 und/oder Aktivierungselement 16 über eine Kommunikation 28 mitgeteilt. Die Kommunikation kann im verbundenen Falle auch von 16 über 14 via 28b erfolgen.

Figur 3b zeigt eine Variante der Erfindung, bei der sich die zur Wahl stehenden Instrumente in einem Rack 28 befinden, welches die den Instrumenten zugeordneten Aktivierungselemente beinhaltet. In diesem Fall kommuniziert die Steuereinrichtung 22 über 28 mit dem Rack, um das gewünschte Werkzeug zu aktivieren. Nur das aktivierte Werkzeug kann vom Nutzer aus dem Rack entnommen werden.

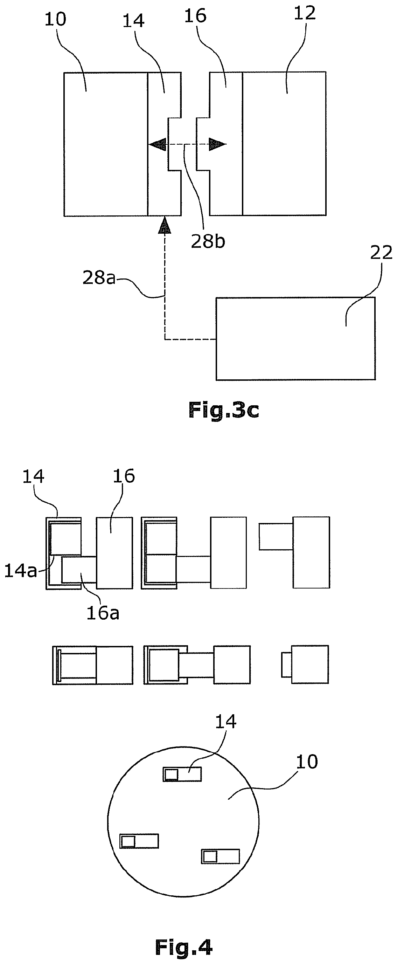

Figur 3c zeigt eine Variante der Erfindung, bei der die Steuereinrichtung nur mit dem Aktivierungselement 14 am Roboter 10 direkt kommuniziert. Um einen sicheren Werkzeugwechsel durchzuführen, teilt die Steuereinrichtung 22 dem Aktivierungselement 14 mit, welches Werkzeug mit dem Roboter verbunden werden soll. Danach kann am Verbindungselement 14 des Roboters 10 nur das Instrument mit dem korrespondierenden Aktivierungselement 16 verbunden werden. Um das korrespondierende Aktivierungselement zu erkennen findet ein Informationsaustausch 28b zwischen dem Aktivierungselement 16 am Instrument und dem Aktivierungselement 14 am Roboter statt (es ist aber keine direkte Kommunikation zwischen dem Instrument und der Steuereinrichtung notwendig).

Im Folgenden werden einige Ausführungsvarianten der Aktivierungselemente ausgeführt.

Eine mögliche Variante, ist die Aktivierung durch einen bestimmten Code, der per Software, elektrischen Signalen oder mechanisch (Schlüssel-Schloss) übermittelt werden kann.

Eine Variante von Figur 3a ist eine korrespondierende Aktivierung durch die Übermittlung eines nur für den gewünschten Zeitpunkt und nur für eine bestimmte Verbindung gültigen Schlüssels (Codes) von der Steuereinrichtung 22 zum Aktivierungselement 14 zum Aktivierungselement 16 am Instrument. Eine Verbindung kommt zustande, wenn durch den Austausch der Schlüssel über die Datenkommunikation 28b eine Übereinstimmung festgestellt wird.

Eine mögliche elektromechanische Variante zeigt Figur 4. Das Aktivierungselement 14 besteht aus einer Vertiefung 14b, in der sich ein Stift 14a befindet. Die Position des Stiftes innerhalb der Vertiefung kann durch eine Steuereinrichtung verändert werden. Dies kann horizontal (Variante a) oder vertikal (Variante b) ausgeführt werden. Das korrespondierende Aktivierungselement 16 hat eine Erhöhung 16a, welche nur bei exakt einer bestimmten Position des Stiftes 14a in die Vertiefung des Aktivierungselements 14 eingeführt werden kann. Nur in diesem Fall kann eine Verbindung hergestellt werden.

Der Stift kann mindestens zwei unterschiedliche bekannte Positionen einnehmen. Es ist aber auch eine quasi stufenlose Verstellbarkeit möglich. Die Anzahl der unterscheidenden Positionen bestimmt die Anzahl der möglichen Verbindungsvarianten zwischen den Aktivierungselementen. Durch Anbringung von mehreren Aktivierungselementen an einer Verbindung 10 kann diese Anzahl weiter erhöht werden. Die zu dieser Ausführung gehörige Kommunikationsvariante entspricht der in Figur 3c, wobei die Kommunikation 28b mechanisch ausgeführt ist.

## Patentansprüche

1. Handhabungseinrichtung, insbesondere Roboter, mit
unterschiedlichen Instrumenten,
einer Aufnahmevorrichtung (34) zur Aufnahme der unterschiedlichen Instrumente (38, 40, 42),
wobei die Aufnahmevorrichtung (34) und die unterschiedlichen Instrumente (38, 40, 42) miteinander kompatible Verbindungselemente (14, 16) aufweisen,
an den Instrumenten (38, 40, 42) vorgesehenen, veränderbaren Indikatoreinrichtungen (44, 46, 48),
einer zusätzlich zu den Indikatoreinrichtungen (44, 46, 48) der Instrumente (38, 40, 42) an der Aufnahmevorrichtung (34) vorgesehenen Indikatoreinrichtung (36), und einer mit den Indikatoreinrichtungen (36, 44, 46, 48) verbundenen Steuereinrichtung (22) zur Veränderung der Indikatoreinrichtungen (36, 44, 46, 48), insbesondere in Abhängigkeit einer Steuervorgabe, wobei zur Indikation einer erforderlichen Verbindung die indikatoreinrichtung (36) der Aufnahmevorrichtung (34) und die Indikatoreinrichtung (44, 46, 48) des geforderten Instruments (38, 40, 42) korrespondierende Indikatoren aufweisen.

2. Handhabungseinrichtung nach Anspruch 1, **dadurch gekennzeichnet, dass** die Indikatoreinrichtungen (36, 44, 46, 68) Leuchtmittel aufweisen.

3. Handhabungseinrichtung nach den Ansprüchen 1 oder 2, **dadurch gekennzeichnet, dass** die Indikatoreinrichtungen (36, 44, 46, 48) ein Display aufweisen.

4. Handhabungseinrichtung nach einem der Ansprüche 1 - 3, **dadurch gekennzeichnet, dass** die Verbindungselemente (14, 16) von der Steuereinrichtung (22) aktivierbare Aktivierungselemente aufweisen, so dass nur bei aktiviertem Aktivierungselement ein Verbinden mit der Aufnahmevorrichtung (34) möglich ist.

5. Handhabungseinrichtung nach einem der Ansprüche 1 - 4, **dadurch gekennzeichnet, dass** in der Steuereinrichtung (22) ein Ablaufplan hinterlegbar ist, in dem die zeitliche Abfolge der Instrumentenwechsel festgelegt ist.

6. Handhabungseinrichtung nach einem der Ansprüche 1 - 5, **dadurch gekennzeichnet, dass** die Steuereinrichtung (22) mit einer Eingabeeinrichtung (28) verbunden ist, um einen gewünschten Instrumentenwechsel einzugeben.

7. Verfahren zum Verbinden von unterschiedlichen Instrumenten (38, 40, 42) mit einer Aufnahmevorrichtung (34) einer Handhabungseinrichtung nach einem der Ansprüche 1 - 6, bei welchem von einer Steuereinrichtung (22) auf Basis eines hinterlegten Ablaufplans und/ oder mittels einer Eingabeeinrichtung (28) eine an dem zu verbindenden Instrument (38, 40, 42) angeordnete Indikatoreinrichtung (44 46, 48) sowie eine an der Aufnahmevorrichtung (34) angeordnete Indikatoreinrichtung (36) aktiviert werden und ein Verbinden dieses Instruments (38, 40, 42) mit der Aufnahmevorrichtung (34) erfolgt.

## Claims

1. A handling device, in particular a robot, comprising
different instruments,
a receiving device (34) for receiving the different instruments (38, 40, 42),
the receiving device (34) and the different instruments (38, 40, 42) comprising mutually compatible connecting elements (14, 16),
changeable indicator devices (44, 46, 48) provided on the instruments (38, 40, 42),
an indicator device (36) provided on the receiving device (34) in addition to the indicator devices (44, 46, 48) of the instruments (38, 40, 42), and
a control device (22) connected to the indicator devices (36, 44, 46, 48) for changing the indicator devices (36, 44, 46, 48), particularly in accordance with preset control data,
wherein, for indicating a required connection, the indicator device (36) of the receiving device (34) and the indicator device (44, 46, 48) of the requested instrument (38, 40, 42) comprise corresponding indicators.

2. The handling device according to claim 1, **characterized in that** the indicator devices (36, 44, 46, 48) comprise illuminants.

3. The handling device according to claim 1 or 2, **characterized in that** the indicator devices (36, 44, 46, 48) comprise a display.

4. The handling device according to any one of claims 1 - 3, **characterized in that** the connecting elements (14, 16) comprise activation elements to be activated by the control unit (22) so that a connection to the receiving device (34) is possible only in the activated state of the activation element.

5. The handling device according to any one of claims 1 - 4, **characterized in that**, in the control unit (22), a workflow plan can be pre-stored in which the temporal order of the changes of instruments is defined.

6. The handling device according to any one of claims 1 - 5, **characterized in that** the control unit (22) is connected to an input device (28) for inputting a desired change of instruments.

7. A method for connecting different instruments (38, 40, 42) to a receiving device (34) of a handling device according to any one of claims 1 - 6, wherein, by means of a control unit (22) and on the basis of a stored workflow plan and/or by use of an input device (28), an indicator device (44, 46, 48) arranged on the instrument (38, 40, 42) to be connected and an indicator device (36) arranged on the receiving device (34) will be activated and said instrument (38, 40, 42) will be connected to the receiving device (34).

## Revendications

1. Mécanisme de manipulation, en particulier un robot, comprenant .
- divers instruments ;
- un dispositif de réception (34) destiné à la réception des divers instruments (38, 40, 42), selon lequel le dispositif de réception (34) et les divers instruments (38, 40, 42) présentent des éléments de liaison (14, 16) qui sont compatibles entre eux ;
- des moyens indicateurs (44, 46, 48) pouvant être modifiés, lesquels sont prévus au niveau des instruments (38, 40, 42) ;
- un moyen indicateur (36) prévu au niveau du dispositif de réception (34), en plus des moyens indicateurs (44, 46, 48) des instruments (38, 40, 42) ; et
- un mécanisme de commande (22) raccordé aux moyens indicateurs (36, 44, 46, 48) et destiné à la modification des moyens indicateurs (36, 44, 46, 48), en particulier en fonction d'une consigne de commande,
selon lequel le moyen indicateur (36) du dispositif de réception (34) et le moyen indicateur (44, 46, 48) de l'instrument (38, 40, 42) requis présentent des indicateurs correspondant à des fins d'indication d'une jonction nécessaire.

2. Mécanisme de manipulation selon la revendication 1, **caractérisé en ce que** les moyens indicateurs (36, 44, 46, 68) présentent des moyens d'éclairage.

3. Mécanisme de manipulation selon les revendications 1 ou 2, **caractérisé en ce que** les moyens indicateurs (36, 44, 46, 48) présentent un affichage.

4. Mécanisme de manipulation selon l'une des revendications 1 à 3, **caractérisé en ce que** les éléments de liaison (14, 16) présentent des éléments d'activation qui peuvent être activés par le mécanisme de commande (22), de telle sorte qu'une jonction avec le dispositif de réception (34) n'est possible que si l'élément d'activation est activé.

5. Mécanisme de manipulation selon l'une des revendications 1 à 4, **caractérisé en ce qu'**un plan de déroulement peut être mémorisé dans le mécanisme de commande (22) et au sein duquel la séquence temporelle du changement d'instrument est déterminée.

6. Mécanisme de manipulation selon l'une des revendications 1 à 5, **caractérisé en ce que** le mécanisme de commande (22) est raccordé à un mécanisme de saisie (28) en vue de saisir un changement d'instrument souhaité.

7. Procédé destiné à la jonction de divers instruments (38, 40, 42) comprenant un dispositif de réception (34) d'un mécanisme de manipulation selon l'une des revendications 1 à 6, pour lequel un moyen indicateur (44, 46, 48) disposé au niveau de l'instrument (38, 40, 42) à raccorder, ainsi qu'un moyen indicateur (36) disposé au niveau du dispositif de réception (34) sont activés par un mécanisme de commande (22) sur la base d'un plan de déroulement enregistré et/ou au moyen d'un mécanisme de saisie (28), permettant de ce fait une jonction de cet instrument (38, 40, 42) avec le dispositif de réception (34).
